# EUROPEAN PATENT SPECIFICATION

(11) **EP 2 766 060 B1**
(45) Date of publication and mention of the grant of the patent: **23.11.2022**
(21) Application number: 12772918.4
(22) Date of filing: 10.10.2012
(51) Int. Cl.: A61L 24/04, A61L 26/00, A61L 24/00

(54) **HEMOSTATIC COMPOSITION**
HÄMOSTATISCHE ZUSAMMENSETZUNG
COMPOSITION HÉMOSTATIQUE

(30) Priority: 11.10.2011 US 201161545939 P
(43) Date of publication of application: 20.08.2014
(60) Divisional application: 22200796.5
(73) Proprietor: Baxter International Inc., Deerfield, IL 60015 (US); Baxter Healthcare SA, 8152 Glattpark (Opfikon) (CH)
(72) Inventor: HEDRICH, Hans Christian, 1020 Vienna (AT); HOEFINGHOFF, Joris, 1110 Vienna (AT)
(74) Representative: SONN Patentanwälte OG
(86) International application number: PCT/EP2012/070054
(87) International publication number: WO 2013/053753

(56) References cited:
- WO-A2-2008/016983
- US-A1- 2002 032 463
- US-A1- 2006 258 560
- US-A1- 2010 318 048
- US-B1- 6 312 725

## Description

### FIELD OF THE INVENTION

The present invention relates to hemostatic compositions and processes for making such compositions.

### BACKGROUND OF THE INVENTION

Hemostatic compositions in dry storage-stable form that comprise biocompatible, biodegradable, dry stable granular material are known e.g. from WO98/008550A or WO 2003/007845A. These products have been successfully applied in the art for hemostasis. Floseal^{®} is an example for a powerful and versatile hemostatic agent consisting of a granular gelatin matrix swollen in a thrombin-containing solution to form a flowable paste.

Since such products have to be applied to humans, it is necessary to provide highest safety standards for quality, storage-stability and sterility of the final products and the components thereof. On the other hand, manufacturing and handling should be made as convenient and efficient as possible.

On the other hand, it has been found that previous hemostatic compositions failed to induce hemostasis at conditions with impaired hemostasis (e.g. after heparinization). It is therefore desired to provide materials and compositions with improved hemostasis. Moreover, a strong adherence of the compositions applied to the tissue is needed when the composition is applied to a wound. It is also desired to provide material with suitable swelling behavior after application to a wound. US 2006/0258560 A1 discloses a tissue sealant composition comprising a crosslinked agent and a synthetic collagen or a synthetic gelatin in a dry state.

It is an object of the present invention to overcome such problems and provide suitable hemostatic compositions with improved adhering properties and methods for making such hemostatic composition. The compositions should also be provided in a convenient and usable manner. The products should preferably be provided in product formats enabling a convenient provision of "ready-to-use" hemostatic compositions, which can be directly applied to an injury without any time consuming reconstitution steps involved.

### SUMMARY OF THE INVENTION

Therefore, the present invention provides a hemostatic composition comprising:
a) a biocompatible polymer in particulate form suitable for use in hemostasis, wherein the biocompatible polymer is a crosslinked gelatin and wherein the particles have a median size of 10 to 1000 µm, and
b) one hydrophilic polymeric component comprising reactive groups, wherein the presence of a hydrophilic polymeric component with nucleophilic reactive groups is excluded; wherein said hydrophilic polymeric component is a hydrophilic crosslinker with electrophilic reactive groups and wherein the biocompatible polymer and the hydrophilic polymeric crosslinker are present in a solid matrix.

The combination of a biocompatible polymer in particulate form with one hydrophilic polymeric component provides a composition with improved hemostatic properties and with improved tissue adherence. This is specifically suitable for wound treatment wherein induction of hemostasis failed, e.g. at conditions with impaired hemostasis (e.g. after heparinization). The compositions according to the present invention improve hemostasis. Furthermore, the compositions according to the present invention show a strong adherence to the tissue when applied to a wound.

Upon contact with bleeding tissue, a crosslinking reaction of the hydrophilic polymeric component with the blood proteins leads to formation of a gel with sealing and hemostatic properties. Crosslinking also occurs to the tissue surface proteins and, depending on the nature of the biocompatible polymer material, may also occur to the biocompatible polymer material. The latter reaction contributes to an improved adhesion of the composition material to the wounded tissue surface.

A further aspect relates to a hemostatic composition for use in the treatment of an injury.

Also provided is a kit for the treatment of an injury, comprising a hemostatic composition as herein disclosed and instructions for use.

The present invention also refers to a method for producing the hemostatic composition according to the invention in a convenient manner allowing the composition to be easily at hand for medical use. The disclosure further relates to a method for delivering a hemostatic composition to a target site in a patient's body, said method comprising delivering a hemostatic composition produced by the process of the present invention to the target site.

However, this method for delivering is not part of the present invention.

According to another aspect, the present disclosure relates to a finished final container obtained by the process according of the present invention containing the present hemostatic composition. The disclosure also relates to a method for providing a ready-to-use hemostatic composition comprising contacting a hemostatic composition produced by the process of the present invention as well as to a kit comprising the finished final container and other means for applying the composition. The compositions according to the present invention are particularly useful for providing hemostasis at bleeding sites, including surgical bleeding sites, traumatic bleeding sites and the like. An exemplary use of the compositions may be in sealing the tissue tract above a blood vessel penetration created for vascular catheterization.

### DESCRIPTION OF SPECIFIC EMBODIMENTS OF THE INVENTION

The present invention provides an improvement in hemostatic compositions. The hemostatic compositions according to the present invention contain biocompatible polymers in particulate form, e.g. granules of a crosslinked gelatin suitable for use in hemostasis (the "hemostatic biocompatible polymer component" or the "hemostatic polymer"). Admixed to this biocompatible polymer for hemostasis is one hydrophilic polymeric component comprising electrophilic reactive groups. According to the present invention, the reactive groups of the polymeric component have retained their reactivity until the composition is brought to the place of clinical action, e.g. onto the wound.

The biocompatible polymers in particulate form suitable for use in hemostasis may include dimensionally isotropic or non-isotropic forms. For example, the biocompatible polymers according to the present invention may be granules or fibers; and may be present in discontinuous structures, for example in powder forms.

According to a preferred embodiment, the biocompatible polymer is liquid absorbing. For example, upon contact with liquids, e.g. aqueous solutions or suspensions (especially a buffer or blood) the polymer takes up the liquid and will display a degree of swelling, depending on the extent of hydration. The material preferably absorbs from about 200% to about 2000%, especially from about 400% to about 1300% water or aqueous buffer by weight, corresponding to a nominal increase in diameter or width of an individual particle of subunit in the range from e.g. approximately 50% to approximately 500%, usually from approximately 50% to approximately 250%. For example, if the (dry) granular particles have a preferred size range of 0.01 mm to 1.5 mm, especially of 0.05 mm to 1 mm, the fully hydrated composition (e.g. after administration on a wound or after contact with an aqueous buffer solution) may have a size range of 0.05 mm to 3 mm, especially of 0.25 mm to 1.5 mm.

The equilibrium swell of preferred biocompatible polymers of the present invention may generally range e.g. from 400% to 1300%, preferably being from 500% to 1100%, depending on its intended use. Such equilibrium swell may be controlled e.g. (for a crosslinked polymer) by varying the degree of crosslinking, which in turn is achieved by varying the crosslinking conditions, such as the type of crosslinking method, duration of exposure of a crosslinking agent, concentration of a crosslinking agent, crosslinking temperature, and the like. Materials having differing equilibrium swell values perform differently in different applications. For example, the ability to inhibit bleeding in a liver divot model was most readily achieved with crosslinked gelatin materials having a swell in the range from 700% to 950%. For a femoral artery plug, lower equilibrium swell values in the range from 500% to 600% were more successful. Thus, the ability to control crosslinking and equilibrium swell allows the compositions of the present invention to be optimized for a variety of uses. In addition to equilibrium swell, it is also important to control the hydration of the material immediately prior to delivery to a target site. Hydration and equilibrium swell are, of course, intimately connected. A material with 0% hydration will be non-swollen. A material with 100% hydration will be at its equilibrium water content. Hydrations between 0% and 100% will correspond to swelling between the minimum and maximum amounts.

According to the present invention, the biocompatible polymer in particulate form suitable for use in hemostasis and the hydrophilic polymeric component are present in a solid matrix.

Biocompatible polymers in particulate form suitable for use in hemostasis may be formed from biologic and non-biologic polymers. Suitable biologic polymers may contain a protein, a polysaccharide, a biologic polymer, a non-biologic polymer; and derivatives and combinations thereof. Suitable proteins include gelatin, collagen, albumin, hemoglobin, fibrinogen, fibrin, casein, fibronectin, elastin, keratin, and laminin; and derivatives and combinations thereof. Particularly preferred is the use of gelatin or soluble non-fibrillar collagen, more preferably gelatin, and exemplary gelatin formulations are set forth below. Other suitable biologic polymers include polysaccharides, such as glycosaminoglycans, starch, cellulose, dextran, hemicellulose, xylan, agarose, alginate and chitosan; and derivatives and combinations thereof. Suitable non-biologic polymers will be selected to be degradable by either of two mechanisms, i.e. (1) break down of the polymeric backbone or (2) degradation of side chains which result in aqueous solubility. Exemplary non-biologic biocompatible polymers suitable for use in hemostasis include synthetics, such as polyacrylates, polymethacrylates, polyacrylamides, polymethacrylamides, polyethyleneimines, polyvinyl resins, polylactide-glycolides, polycaprolactones, and polyoxyethlenes; and derivatives and combinations thereof. Also combinations of different kinds of polymers are possible (e.g. proteins with polysaccharides, proteins with non-biologic hydrogel-forming polymers, etc.). Preferred hemostatic polymers comprise amino-groups, specifically if the hydrophilic polymeric component has reactive groups which react with amino-groups upon administration (e.g. in the wound environment).

"A derivative thereof" includes any chemically modified polymer, such as e.g. a crosslinked polymer.

Preferred hemostatic polymers comprise nucleophilic groups, such as e.g. amino-groups, specifically if the hydrophilic polymeric component has reactive groups which react with amino-groups upon administration (e.g. in the wound environment).

According to the present invention, the biocompatible polymer is crosslinked gelatin.

A non-crosslinked polymer may be crosslinked in any manner suitable to reconstitute, e.g. to form a suitable hydrogel base of the hemostatic polymer. For example, polymeric molecules may be crosslinked using bi- or poly-functional crosslinking agents which covalently attach to two or more polymer molecules chains. Exemplary bifunctional crosslinking agents include aldehydes, epoxides, succinimides, carbodiimides, maleimides, azides, carbonates, isocyanates, divinyl sulfone, alcohols, amines, imidates, anhydrides, halides, silanes, diazoacetate, aziridines, and the like. Alternatively, crosslinking may be achieved by using oxidizers and other agents, such as periodates, which activate side-chains or moieties on the polymer so that they may react with other side-chains or moieties to form the crosslinking bonds. An additional method of crosslinking comprises exposing the polymers to radiation, such as gamma radiation, to activate the polymer chains to permit crosslinking reactions. Dehydrothermal crosslinking methods may also be suitable. Preferred methods for crosslinking gelatin molecules are described below.

The biocompatible hemostatic polymer - once applied to a wound - forms an efficient matrix which can form a barrier for blood flow. Specifically the swelling properties of the hemostatic polymer can make it an effective mechanical barrier against bleeding and re-bleeding processes.

In a preferred embodiment, the hemostatic compositions according to the present invention are provided or used as granular preparations.

The biocompatible polymer suitable for use in hemostasis is in particulate form, preferably a granular material. This granular material can rapidly swell when exposed to a fluid (i.e. the diluent) and in this swollen form is capable of contributing to a flowable paste that can be applied to a bleeding site. The biocompatible polymer, i.e. crosslinked gelatin, may be provided as a film which can then be milled to form a granular material. Most of the particles contained in this granular material (e.g. more than 90% w/w) have particle sizes of 100 to 1000 µm, especially 50 to 700 µm.

The biocompatible polymer in particulate form suitable for use in hemostasis is a crosslinked gelatin. Dry crosslinked gelatin powder can be prepared to re-hydrate rapidly if contacted with a pharmaceutically acceptable diluent. The gelatin granules, especially in the form of a gelatin powder, preferably comprise relatively large particles, also referred to as fragments or sub-units, as described in WO98/08550A and WO2003/007845A. The (median) particle size will be the range from 10 to 1000 µm, preferably from 50 to 700 µm. The dry compositions will also display a significant "equilibrium swell" when exposed to an aqueous re-hydrating medium (= diluents, also referred to as reconstitution medium or re-hydration medium). Preferably, the swell will be in the range from 400% to 1000%. "Equilibrium swell" may be determined by subtracting the dry weight of the gelatin hydrogel powder from its weight when fully hydrated and thus fully swelled. The difference is then divided by the dry weight and multiplied by 100 to give the measure of swelling. The dry weight should be measured after exposure of the material to an elevated temperature for a time sufficient to remove substantially all residual moisture, e.g., two hours at 120°C. The equilibrium hydration of the material can be achieved by immersing the dry material in a pharmaceutically acceptable diluent, such as aqueous saline, for a time period sufficient for the water content to become constant, typically for from 18 to 24 hours at room temperature.

According to a preferred embodiment, the pharmaceutically acceptable diluent further comprises thrombin, preferably 10 to 1000 I.U. thrombin/ml, especially 250 to 700 I.U. thrombin/ml. Preferably, the hemostatic composition in this ready to use form contains 10 to 100.000 International Units (I.U.) of thrombin, more preferred 100 to 10.000 I.U., especially 500 to 5.000 I.U.. The thrombin concentration in the ready-to-use composition is preferably in the range of 10 to 10.000 I.U., more preferred of 50 to 5.000 I.U., especially of 100 to 1.000 I.U./ml. The diluent is used in an amount to achieve the desired end-concentration in the ready-to-use composition. The thrombin preparation may contain other useful component, such as ions, buffers, excipients, stabilizers, etc..

Thrombin (or any other coagulation inducing agent, such as a snake venom, a platelet activator, a thrombin receptor activating peptide and a fibrinogen precipitating agent) can be derived from any thrombin preparation which is suitable for use in humans (i.e. pharmaceutically acceptable). Suitable sources of thrombin include human or bovine blood, plasma or serum (thrombin of other animal sources can be applied if no adverse immune reactions are expected) and thrombin of recombinant origin (e.g. human recombinant thrombin); autologous human thrombin can be preferred for some applications.

Exemplary methods for producing crosslinked gelatins are as follows. Gelatin is obtained and suspended in an aqueous solution to form a non-crosslinked hydrogel, typically having a solids content from 1% to 70% by weight, usually from 3% to 10% by weight. The gelatin is crosslinked, typically by exposure to either glutaraldehyde (e.g., 0.01% to 0.05% w/w, overnight at 0°C. to 15°C in aqueous buffer), sodium periodate (e.g., 0.05 M, held at 0°C. to 15°C. for 48 hours) or 1-ethyl-3-(3-dimethylaminopropyl) carbodiimide ("EDC") (e.g., 0.5% to 1.5% w/w overnight at room temperature), or by exposure to about 0.3 to 3 megarads of gamma or electron beam radiation. Alternatively, gelatin particles can be suspended in an alcohol, preferably methyl alcohol or ethyl alcohol, at a solids content of 1% to 70% by weight, usually 3% to 10% by weight, and crosslinked by exposure to a crosslinking agent, typically glutaraldehyde (e.g., 0.01% to 0.1% w/w, overnight at room temperature). In the case of aldehydes, the pH should be held from about 6 to 11, preferably from 7 to 10. When crosslinking with glutaraldehyde, the crosslinks are formed via Schiff bases which may be stabilized by subsequent reduction, e.g., by treatment with sodium borohydride. After crosslinking, the resulting granules may be washed in water and optionally rinsed in an alcohol, and dried. The resulting dry powders may then be provided in the final container as described herein.

Preferably, the biocompatible polymer is provided in a dry granular form for producing the hemostatic compositions according to the present invention. A "dry granular preparation of a biocompatible polymer" according to the present invention is known e.g. from WO 98/08550 A. Preferably, the polymer is a biocompatible, biodegradable dry stable granular material.

The dry polymer according to the present invention is provided with particle sizes of 10 to 1000 µm. Usually, the polymer particles have a mean particle diameter ("mean particle diameter" is the median size as measured by laser diffractometry; "median size" (or mass median particle diameter) is the particle diameter that divides the frequency distribution in half; fifty percent of the particles of a given preparation have a larger diameter, and fifty percent of the particles have a smaller diameter) from 10 to 1000 µm, especially 50 to 700 µm (median size). Applying larger particles is mainly dependent on the medical necessities; particles with smaller mean particle diameters are often more difficult to handle in the production process. The dry polymer is therefore provided in granular form. Although the terms powder and granular (or granulates) are sometimes used to distinguish separate classes of material, powders are defined herein as a special sub-class of granular materials. In particular, powders refer to those granular materials that have the finer grain sizes, and that therefore have a greater tendency to form clumps when flowing. Granules include coarser granular materials that do not tend to form clumps except when wet. For the present application the particles used are those which can be coated by suitable coating techniques Particle size of the polymer granules according to the present invention can therefore easily be adapted and optimized to a certain coating technique by the necessities of this technique.

The hydrophilic polymeric component (also referred to as "reactive hydrophilic component" or "hydrophilic (polymeric) crosslinker") of the hemostatic composition according to the present invention is a hydrophilic crosslinker which is able to react with its reactive groups once the hemostatic composition is applied to a patient (e.g. to a wound of a patient or another place where the patient is in need of a hemostatic activity). Therefore it is important for the present invention that the reactive groups of the polymeric component are reactive when applied to the patient. It is therefore necessary to manufacture the hemostatic composition according to the present invention so that the reactive groups of the polymeric component which should react once they are applied to a wound are retained during the manufacturing process.

This can be done in various ways. For example, usual hydrophilic polymeric components have reactive groups which are susceptible to hydrolysis after contact with water. Accordingly, premature contact with water or aqueous liquids has to be prevented before administration of the hemostatic composition to the patient, especially during manufacture. However, processing of the hydrophilic polymeric component during manufacturing may be possible also in an aqueous medium at conditions where the reactions of the reactive groups are inhibited (e.g. at a low pH). If the hydrophilic polymeric components can be melted, the melted hydrophilic polymeric components can be sprayed or printed onto the matrix of the biopolymer. It is also possible to mix a dry form (e.g. a powder) of the hydrophilic polymeric component with a dry form of the biocompatible polymer suitable for use in hemostasis. If necessary, then an increase of the temperature can be applied to melt the sprinkled hydrophilic polymeric component to the biocompatible polymer suitable for use in hemostasis to achieve a permanent coating of the hemostatic composition. Alternatively, these hydrophilic polymeric components can be taken up into inert organic solvents (inert vis-à-vis the reactive groups of the hydrophilic polymeric components) and brought onto the matrix of the biomaterial. Examples of such organic solvents are dry ethanol, dry acetone or dry dichloromethane (which are e.g. inert for hydrophilic polymeric components, such as NHS-ester substituted PEGs).

The term "one hydrophilic polymeric component comprising reactive groups" means that the presence of a second or further hydrophilic polymeric component with nucleophilic reactive groups is excluded in a hemostatic composition according to the present invention.

In a preferred embodiment the hydrophilic polymer component is a single hydrophilic polymer component and is a polyalkylene oxide polymer, preferably a PEG comprising polymer. The reactive groups of this reactive polymer are electrophilic groups.

The reactive hydrophilic component may be a multi-electrophilic polyalkylene oxide polymer, e.g. a multi-electrophilic PEG. The reactive hydrophilic component can include two or more electrophilic groups, preferably a PEG comprising two or more reactive groups selected from succinimidylesters (-CON(COCH₂)₂), aldehydes (-CHO) and isocyanates (-N=C=O), e.g. a component as disclosed in the WO2008/016983 A and one of the components of the commercially available ones under the trademark CoSeal^{®}.

Preferred electrophilic groups of the hydrophilic polymeric crosslinker according to the present invention are groups reactive to the amino-, carboxy-, thiol- and hydroxy- groups of proteins, or mixtures thereof.

Preferred amino group-specific reactive groups are NHS-ester groups, imidoester groups, aldehyde-groups, carboxy-groups in the presence of carbodiimides, isocyanates, or THPP (beta-[Tris(hydroxymethyl)phosphino] propionic acid), especially preferred is Pentaerythritolpoly(ethyleneglycol)ether tetrasuccinimidyl glutarate (= Pentaerythritol tetrakis[1-1'-oxo-5'-succinimidylpentanoate-2-poly-oxoethyleneglycole]ether (= an NHS-PEG with MW 10,000).

Preferred carboxy-group specific reactive groups are amino-groups in the presence of carbodiimides.

Preferred thiol group-specific reactive groups are maleimides or haloacetyls.

Preferred hydroxy group-specific reactive group is the isocyanate group.
The reactive groups on the hydrophilic crosslinker may be identical (homofunctional) or different (heterofunctional). The hydrophilic polymeric component can have two reactive groups (homobifunctional or heterobifunctional) or more (homo/hetero-trifunctional or more).

In special embodiments the material is a synthetic polymer, preferably comprising PEG. The polymer can be a derivative of PEG comprising active side groups suitable for crosslinking and adherence to a tissue.

By the reactive groups the hydrophilic reactive polymer has the ability to crosslink blood proteins and also tissue surface proteins. Crosslinking to the biomaterial is also possible.

The multi-electrophilic polyalkylene oxide may include two or more succinimidyl groups. The multi-electrophilic polyalkylene oxide may include two or more maleimidyl groups.

Preferably, the multi-electrophilic polyalkylene oxide is a polyethylene glycol or a derivative thereof.

In a most preferred embodiment the hydrophilic polymeric component is pentaerythritolpoly(ethyleneglycol)ether tetrasuccinimidyl glutarate (=COH102, also pentaerythritol tetrakis[1-1'-oxo-5'-succinimidylpentanoate-2-poly-oxoethyleneglycole]ether).

The hydrophilic polymeric component is a hydrophilic crosslinker. According to a preferred embodiment, this crosslinker has more than two reactive groups for crosslinking ("arms"), for example three, four, five, six, seven, eight, or more arms with reactive groups for crosslinking. For example, NHS-PEG-NHS is an effective hydrophilic crosslinker according to the present invention. However, for some embodiments, a 4-arm polymer (e.g. 4-arms-p-NP-PEG) may be more preferred; based on the same rationale, an 8-arm polymer (e.g. 8-arms-NHS-PEG) may even be more preferred for those embodiments where multi-reactive crosslinking is beneficial. Moreover, the hydrophilic crosslinker according to the present invention is a polymer, i.e. a large molecule (macromolecule) composed of repeating structural units which are typically connected by covalent chemical bonds. The hydrophilic polymer component according to the present invention should have a molecular weight of at least 1000 Da (to properly serve as crosslinker in the hemostatic composition according to the present invention); preferably the crosslinking polymers according to the present invention has a molecular weight of at least 5000 Da, especially of at least 8000 Da.

For some hydrophilic crosslinkers, the presence of basic reaction conditions (e.g. at the administration site) is preferred or necessary for functional performance (e.g. for a faster crosslinking reaction at the administration site). For example, carbonate or bicarbonate ions (e.g. as a buffer with a pH of 7.6 or above, preferably of 8.0 or above, especially of 8.3 and above) may be additionally provided at the site of administration (e.g. as a buffer solution or as a fabric or pad soaked with such a buffer), so as to allow an improved performance of the hemostatic composition according to the present invention or to allow efficient use as a hemostatic and/or wound adherent material.

The reactivity of the hydrophilic polymeric component (which, as mentioned, acts as a crosslinker) in the composition according to the present invention is retained in the composition. This means that the reactive groups of the crosslinker have not yet reacted with the hemostatic composition and are not hydrolyzed by water (or at least not in a significant amount which has negative consequences on the hemostatic functionality of the present compositions). This can be achieved by combining the hemostatic polymer with the hydrophilic crosslinker in a way which does not lead to reaction of the reactive groups of the crosslinker with the hemostatic polymer or with water. Usually, this includes the omitting of aqueous conditions (or wetting), especially wetting without the presence of acidic conditions (if crosslinkers are not reactive under acidic conditions). This allows the provision of reactive hemostatic materials.

According to a specifically preferred hemostatic composition of the invention, the biocompatible polymer is crosslinked gelatin and the hydrophilic polymeric component is pentaerythritolpoly(ethyleneglycol)ether tetrasuccinimidyl glutarate.

Preferred ratios of the biocompatible polymer to hydrophilic polymeric component in the hemostatic composition according to the present invention are from 0.1 to 50 % w/w, preferably from 5 to 40 %w/w.

The present hemostatic composition is provided in a solid matrix, and the use of collagen in (or as) the solid matrix is specifically preferred. An advantage of the hemostatic composition being provided in solid form is that such solid forms may be separable by mechanical means. This allows specific dimensioning of the hemostatic composition at the place of use, e.g. during surgery immediately before or during administration.

The "solid matrix" according to the present invention forms - together with the biocompatible polymer and the hydrophilic polymeric component - a solid form of the composition according to the present invention which may retain its 3-dimensional form in a robust manner. Accordingly, the solid matrix also acts as a "matrix forming component" for the hemostatic ingredients of the composition according to the present invention.

Preferred solid matrices according to the present invention provide a porous structure and/or a fibrous network which allows liquids (e.g. blood, a buffer or reactive components) to enter the matrix. These matrices according to the present invention include woven and nonwoven materials. They may show a continuous phase or be present in discontinuous phases (e.g. multi-layered). Preferably, the solid matrix according to the present invention is a permeable matrix. It may be provided as a temporary (e.g. biodegradable or permanent matrix. According to a preferred embodiment, the solid matrix comprises nucelophilic groups, such as amino groups.

The present hemostatic composition is preferably provided in lyophilized form. As a lyophilisate, transport and storage properties are significantly improved which enables the use of the present invention also in places where steady cooling cannot be guaranteed.

Further components may be present in the hemostatic composition according to the present invention. According to preferred embodiments, the hemostatic compositions according to the present invention may further comprise a substance selected from the group consisting of antifibrinolytic, procoagulant, platelet activator, antibiotic, vasoconstrictor, dye, growth factors, bone morphogenetic proteins and pain killers.

According to another aspect, the present invention relates to a hemostatic composition according to the present invention for use in the treatment of an injury selected from the group consisting of a wound, a hemorrhage, damaged tissue, bleeding tissue and/or bone defect.

According to another aspect, the present invention provides a kit for the treatment of an injury selected from the group consisting of a wound, a hemorrhage, damaged tissue and/or bleeding tissue comprising
a) a hemostatic composition according to the present invention; and
b) instructions for use

The present invention also relates to a method for producing a hemostatic composition according to the present invention comprising the step of mixing, preferably blending, a biocompatible polymer suitable for use in hemostasis and one hydrophilic polymeric component comprising reactive groups with a matrix-forming component under conditions wherein the reactivity of the polymeric component is retained and drying said composition, preferably by freeze-drying, wherein the biocompatible polymer is a crosslinked gelatin and wherein the hydrophilic polymeric component is a hydrophilic crosslinker with electrophilic reactive groups.

According to another aspect, the present disclosure also provides a method for delivering a hemostatic composition according to the invention to a target site in a patient's body, said method comprising delivering a hemostatic composition produced by the process according to the present invention to the target site. However, this method for delivering is not part of the present invention. Although in certain embodiments, also the dry composition can be directly applied to the target site (and, optionally be contacted with the pharmaceutically acceptable diluent a the target site, if necessary), it is preferred to contact the dry hemostatic composition with a pharmaceutically acceptable diluent before administration to the target site, so as to obtain a hemostatic composition in a wetted form, especially a hydrogel form.

The present disclosure also refers to a finished final container obtained by the process according to the present invention. This finished container contains the combined components in a sterile, storage-stable and marketable form. The final container can be any container suitable for housing (and storing) pharmaceutically administrable compounds.

The invention is further described in the examples below, yet without being restricted thereto.

### EXAMPLES

### Example 1: Preparation of bovine collagen suspension

50g of sliced bovine corium were dispersed in 500ml of a 2M NaOH solution and stirred approx. 90 minutes at 25°C. The corium was sieved out and rinsed with distilled H₂O until effluent H₂O reached a pH of about 8.0. The washed corium slices were re-suspended in H₂O and the pH was adjusted with HCI to approx. 2.0. A suspension obtained was stirred overnight at approx. 25°C and a collagen solution was obtained. A solution obtained was cooled to 5°C and the pH was adjusted with NaOH to neutral. Collagen precipitation was carried out overnight by keeping the solution at 18°C without stirring. Precipitated collagen obtained was separated by filtration. The collagen concentration of the material obtained was determined by gravimetry. Optionally a chemical crosslinking with glutaraldehyde may be carried out in that a 1% aq. collagen suspension was prepared and 5000ppm of glutaraldehyde are added at 12°C. A suspension obtained was stirred overnight. Crosslinked collagen obtained was filtered and washed with H₂O. The collagen concentration of the material obtained was determined by gravimetry as described above.

### Example 2: Preparation of a hemostatic, NHS-PEG impregnated solid form of crosslinked gelatin particles embedded in a loose collagen network.

25.5ml of a suspension containing 69.1mg/cm³ crosslinked gelatin particles (Floseal^{™}, Baxter) in an aqueous, acidic solution (pH 3.0, CH₃COOH) containing 1.9mg/cm³ of bovine collagen (prepared according to example 1) and 19.8 mg/cm³ NHS-PEG (COH102) were prepared, filled into 9x7cm PET-trays and lyophilized. A composition comprising gelatin and NHS-PEG in a solid collagen matrix was obtained ("cake").

Each "cake" was placed together with a sachet containing desiccant into a gas-impermeable pouch, the pouch was sealed and gamma-sterilized at 25kGy.

### Example 3: In vivo data

On an anesthetized, heparinized pig (1.5 x ACT) a liver lobe was punctured by stabbing with a tool containing two perpendicular sharp blades (2 cm width each). Thus a cross-shaped perforation of the liver lobe was obtained. The pouch containing the hemostatic solid form according to Example 2 was opened and the necessary amount of material was broken from the preparation. The material was stuffed with the aid of surgical gloves into the bleeding wound. The wound containing the hemostatic material was compressed for 3 minutes from both ends of the perforation using surgical gloves. The strong bleeding was stopped by this treatment.

## Claims

1. Hemostatic composition comprising:
a) a biocompatible polymer in particulate form suitable for use in hemostasis, wherein the biocompatible polymer is a crosslinked gelatin and wherein the particles have a median size of 10 to 1000 µm, and
b) one hydrophilic polymeric component comprising reactive groups, wherein the presence of a hydrophilic polymeric component with nucleophilic reactive groups is excluded;
wherein said hydrophilic polymeric component is a hydrophilic crosslinker with electrophilic reactive groups and wherein the biocompatible polymer and the hydrophilic polymeric crosslinker are present in a solid matrix.

2. Hemostatic composition according to claim 1, wherein the hydrophilic polymer component is a polyalkylene oxide polymer, preferably a PEG comprising polymer, more preferred a multi-electrophilic polyalkylene oxide polymer, especially a multi-electrophilic PEG, such as pentaerythritolpoly(ethyleneglycol)ether tetrasuccinimidyl glutarate.

3. Hemostatic composition according to claim 1 or 2, wherein said hydrophilic polymeric component with reactive groups is a polyethylene glycol (PEG), preferably a PEG comprising two or more reactive groups selected from succinimidylesters (-CON(COCH₂)₂), aldehydes (-CHO) and isocyanates (-N=C=O).

4. Hemostatic composition according to any one of claims 1 to 3, wherein the biocompatible polymer is crosslinked gelatin and the hydrophilic polymeric component is pentaerythritolpoly(ethyleneglycol)ether tetrasuccinimidyl glutarate.

5. Hemostatic composition according to any one of claims 1 to 4, wherein the solid matrix is collagen.

6. Hemostatic composition according to any one of claims 1 to 5 for use in the treatment of an injury selected from the group consisting of a wound, a hemorrhage, damaged tissue, bleeding tissue and/or bone defects.

7. Kit comprising
a) a hemostatic composition according to any one of claims 1 to 5; and
b) instructions for use.

8. Method for producing a hemostatic composition according to any one of claims 1 to 5 comprising the step of mixing a biocompatible polymer suitable for use in hemostasis and one hydrophilic polymeric component comprising reactive groups with a matrix-forming component under conditions wherein the reactivity of the polymeric component is retained and drying said composition, preferably by freeze-drying, wherein the biocompatible polymer is a crosslinked gelatin and wherein the hydrophilic polymeric component is a hydrophilic crosslinker with electrophilic reactive groups.

9. Method according to claim 8, wherein the biocompatible polymer is mixed with an acidic solution comprising the matrix-forming component, especially collagen, and the hydrophilic polymeric component, especially pentaerythritolpoly(ethyleneglycol)ether tetrasuccinimidyl glutarate.

## Patentansprüche

1. Hämostatische Zusammensetzung, aufweisend:
a) ein biokompatibles Polymer in Teilchenform, das zur Anwendung bei der Hämostase geeignet ist, wobei das biokompatible Polymer eine vernetzte Gelatine ist und wobei die Teilchen eine mittlere Größe von 10 bis 1 000 µm haben, und
b) eine hydrophile polymere Komponente, die reaktive Gruppen aufweist, wobei die Gegenwart einer hydrophilen polymeren Komponente mit nukleophilen reaktiven Gruppen ausgeschlossen ist;
wobei die hydrophile polymere Komponente ein hydrophiler Vernetzer mit elektrophilen reaktiven Gruppen ist und wobei das biokompatible Polymer und der hydrophile polymere Vernetzer in einer festen Matrix vorliegen.

2. Hämostatische Zusammensetzung nach Anspruch 1, wobei die hydrophile polymere Komponente ein Polyalkylenoxidpolymer ist, vorzugsweise ein PEG umfassendes Polymer, besonders bevorzugt ein mehrfach elektrophiles Polyalkylenoxidpolymer, insbesondere ein mehrfach elektrophiles PEG, wie Pentaerythritolpoly(ethylenglykol)ether-Tetrasuccinimidylglutarat.

3. Hämostatische Zusammensetzung nach Anspruch 1 oder 2, wobei die hydrophile polymere Komponente mit reaktiven Gruppen ein Polyethylenglykol (PEG) ist, vorzugsweise ein PEG, aufweisend zwei oder mehr reaktive Gruppen, ausgewählt aus Succinimidylestern (-CON(COCH₂)₂), Aldehyden (-CHO) und Isocyanaten (-N=C=O).

4. Hämostatische Zusammensetzung nach einem der Ansprüche 1 bis 3, wobei das biokompatible Polymer vernetzte Gelatine ist und die hydrophile polymere Komponente Pentaerythritolpoly(ethylenglykol)ether-Tetrasuccinimidylglutarat ist.

5. Hämostatische Zusammensetzung nach einem der Ansprüche 1 bis 4, wobei die feste Matrix Kollagen ist.

6. Hämostatische Zusammensetzung nach einem der Ansprüche 1 bis 5 zur Anwendung bei der Behandlung einer Verletzung ausgewählt aus der Gruppe bestehend aus einer Wunde, einer Blutung, geschädigtem Gewebe, blutendem Gewebe und/oder Knochendefekten.

7. Kit, aufweisend
**a)** eine hämostatische Zusammensetzung nach einem der Ansprüche 1 bis 5 und
**b)** eine Gebrauchsanweisung.

8. Verfahren zur Herstellung einer hämostatischen Zusammensetzung nach einem der Ansprüche 1 bis 5, aufweisend den Schritt des Mischens eines biokompatiblen Polymers, das zur Anwendung bei der Hämostase geeignet ist, und einer hydrophilen polymeren Komponente aufweisend reaktive Gruppen mit einer matrixbildenden Komponente unter Bedingungen, unter denen die Reaktivität der polymeren Komponente beibehalten wird, und des Trocknens der Zusammensetzung, vorzugsweise durch Gefriertrocknen, wobei das biokompatible Polymer eine vernetzte Gelatine ist und wobei die hydrophile polymere Komponente ein hydrophiler Vernetzer mit elektrophilen reaktiven Gruppen ist.

9. Verfahren nach Anspruch 8, wobei das biokompatible Polymer mit einer sauren Lösung gemischt wird, welche die matrixbildende Komponente, insbesondere Kollagen, und die hydrophile polymere Komponente, insbesondere Pentaerythritolpoly(ethylenglykol)ether-Tetrasuccinimidylglutarat, aufweist.

## Revendications

1. Composition hémostatique comprenant :
a) un polymère biocompatible dans une forme particulaire utilisable dans une hémostase, où le polymère biocompatible est une gélatine réticulée et où les particules présentent une taille moyenne de 10 à 1 000 µm, et
b) un constituant polymère hydrophile comprenant des groupes réactifs, où la présence d'un constituant polymère hydrophile avec des groupes réactifs nucléophiles est exclue ;
où ledit constituant polymère hydrophile est un réticulant hydrophile avec des groupes réactifs électrophiles et où le polymère biocompatible et le réticulant polymère hydrophile sont présents dans une matrice solide.

2. Composition hémostatique selon la revendication 1, où le constituant polymère hydrophile est un polymère de poly(oxyde d'alkylène), de préférence un polymère comprenant du PEG, encore mieux un polymère de poly(oxyde d'alkylène) multi-électrophile, particulièrement un PEG multi-électrophile, tel que le pentaérythritolpoly(éthylèneglycol)-éther glutarate de tétrasuccinimidyle.

3. Composition hémostatique selon la revendication 1 ou 2, où ledit constituant polymère hydrophile avec des groupes réactifs est un polyéthylène glycol (PEG), de préférence un PEG comprenant deux groupes réactifs ou plus choisis parmi des esters succinimidyliques (-CON(COCH₂)₂), aldéhydes (-CHO) et isocyanates (-N=C=O).

4. Composition hémostatique selon l'une quelconque des revendications 1 à 3, où le polymère biocompatible est de la gélatine réticulée et le constituant polymère hydrophile est le pentaérythritolpoly(éthylèneglycol)éther glutarate de tétrasuccinimidyle.

5. Composition hémostatique selon l'une quelconque des revendications 1 à 4, où la matrice solide est le collagène.

6. Composition hémostatique selon l'une quelconque des revendications 1 à 5 pour utilisation dans le traitement d'une lésion choisie dans le groupe consistant en une plaie, une hémorragie, un tissu endommagé, un tissu saignant et/ou des défauts osseux.

7. Kit comprenant
a) une composition hémostatique selon l'une quelconque des revendications 1 à 5 ; et
b) des instructions pour l'utilisation.

8. Procédé pour la production d'une composition hémostatique selon l'une quelconque des revendications 1 à 5 comprenant l'étape de mélange d'un polymère biocompatible utilisable dans une hémostase et d'un constituant polymère hydrophile comprenant des groupes réactifs avec un constituant formant une matrice dans des conditions dans lesquelles la réactivité du constituant polymère est conservée et de séchage de ladite composition, de préférence par lyophilisation, où le polymère biocompatible est une gélatine réticulée et où le constituant polymère hydrophile est un réticulant hydrophile avec des groupes réactifs électrophiles.

9. Procédé selon la revendication 8, où le polymère biocompatible est mélangé avec une solution acide comprenant le constituant formant une matrice, particulièrement du collagène, et le constituant polymère hydrophile, particulièrement le pentaérythritol-poly(éthylèneglycol)éther glutarate de tétrasuccinimidyle.
